# EUROPEAN PATENT APPLICATION

(11) **EP 1 442 725 A2**
(43) Date of publication of application: **04.08.2004**
(21) Application number: 04250220.3
(22) Date of filing: 16.01.2004
(51) Int. Cl.: A61F 2/30

(54) **Wear surface for metal-on-metal articulation**

(30) Priority: 24.01.2003 US 350700
(71) Applicant: Biomet, Inc., Warsaw, Indiana 46581 (US)
(72) Inventor: Schroeder, David Wayne, Winona Lake, Indiana 46590 (US)
(74) Representative: Giles, Ashley Simon

(57) **Abstract**

A work hardened articulating surface for anatomical prosthetics. Articulating surfaces of prosthetics are work hardened to decrease the wear and increase the longevity of the prosthetic after implantation. The articulating surface can be hardened in any number of ways, for example the articulating surface can be formed from stock material that has been work hardened throughout or the articulating surface can be work hardened prior to final finishing of the prosthetic component. Regardless, the hardened articulating surface reduces wear after implantation while eliminating the need for a separate bearing component.

## Description

### TECHNICAL FIELD

The disclosure relates to bearing or articulating components in prosthetics, and particularly relates to metal on metal bearing surfaces for use in a prosthetic.

### BACKGROUND

Modern medical interventions enable many portions of the anatomy to be replaced by prosthetic devices, including load bearing or articulating joints. Exemplary bearing joints include the ball and socket of the hip joint and the shoulder joint. Although these are exemplary bearing joints, many other joints, which also have articulating surface, may also be replaced such as the flanges or knee joints.

Often, the bearing joints are replaced with allograft or xenograft prosthetic materials that are at least as hard as the natural bone. A bearing is often placed between the harder portions in a bearing prosthetic. The bearing can include an ultra-high molecular weight polyethylene (UHMWPE) bearing surface. The UHMWPE provides a substantially smooth and cushioned bearing surface between the hard articulation surfaces of the prosthetic. The harder surfaces articulate on the bearing during articulation. This generally increases the wear life of the prosthetic joint.

Alternatively, the articulating portions of a prosthetic may include metal-on-metal (MOM) bearing surfaces. In this case rather than including a separate bearing, such as the UHMWPE, no separate bearing is included. Two surfaces, generally formed of metal, form the surfaces which are the bearing surfaces of the articulating prosthetic. These MOM bearing components have the advantage of being able to carry increased forces without deforming in the joint. Nevertheless, the MOM prosthetics may encounter greater wear because two hardened surfaces continually articulate against one another.

MOM bearing components have been augmented by coating or chemically altering surface on the articulating region of the prosthetic. These hard coatings, include, for example, ceramic coatings that are either deposited or formed on the exterior of the articulating surfaces. If the articulating surfaces include a hardened coating then wear may be substantially reduced. Forming or depositing the coating on the prosthetics is generally very difficult. Moreover, forming a strong adhesion of the coating to the prosthetic for the life of the prosthetic can also be difficult. Adding the coating also requires additional manufacturing steps and is particularly expensive.

Prosthetic devices have also been hardened for strength and longevity, particularly in modular prosthetics. The fully formed prosthetic device is cold or warm worked after being formed to induce internal stresses or transformations. These stresses increase the strength and longevity of the prosthetic. Such methods include U.S. Patent No. 6,067,701 entitled, "Method for Forming a Work Hardened Modular Component Connector," and U.S. Patent Application No. 10/231,944 entitled "Method for Controlling Residual Stress in Prosthetics," both incorporated herein by reference. Work hardening is performed to strengthen a prosthetic portion. These include the stem or a taper that interconnects two portions of a modular prosthetic.

### SUMMARY OF THE DISCLOSURE

A metal on metal bearing prosthetic having articulating surfaces formed of metal. No additional bearing component is included between the articulating portions of the prosthetic. The articulating surfaces of the bearing prosthetic are hardened to increase longevity and decrease possible wear debris during articulation of the prosthetic. The articulating or bearing surfaces of the prosthetic are generally hardened to at least about 45 Rockwell hardness. The bearing surfaces may be hardened either before or after forming the final prosthetic. For example, an entire stock portion of material from which the prosthetic is to be formed may be hardened to a selected hardness. Alternatively, the surfaces may be hardened after rough forming the prosthetic device. After hardening the particular surface, it may then be finished without destroying the hardened surface.

One of the various embodiments includes a prosthetic device for implantation to replace a natural joint. The prosthetic has an articulating region. Moreover the prosthetic includes a first metal prosthetic portion having a first articulating surface a second metal prosthetic portion having a second articulating surface. The first articulating surface and the second articulating surface each include at least about a 45 Rockwell hardness. The first prosthetic portion and the second prosthetic portion may be implanted in the natural joint so that the first articulating surface articulates with the second articulating surface. Also the prosthetic component does not produce significant wear within the prosthetic.

A second of the various embodiments includes a prosthetic for implantation into a body. The prosthetic includes a first member defining a first articulating surface and the first member also defines a first hardness region having a first transition zone at least 0.25 mm below the first articulating surface. A second member defines a second articulating surface adapted to form an articulating region with the first articulating surface. The second member defines a second hardness region having a second transition zone at least 0.25 mm below the second articulating surface. The first member and the second member are implanted in the body so that the first articulating surface articulates with the second articulating surface during functioning of the prosthetic device.

A third of the various embodiments includes a prosthetic to replace a natural joint, the natural joint including a first articulating portion and a second articulating portion. The prosthetic includes a first component adapted to replace the first articulating portion and a second component adapted to replace the second articulating portion. The first component and the second component each include an articulating surface to substantially replace the natural joint after implantation. The first component and the second component are formed from a material having a hardness greater than about 45 Rockwell hardness. The articulating surface of each of the first component and the second component include the hardness of the material.

A fourth of the various embodiments includes a method of forming a prosthetic to replace a natural joint, the prosthetic including a first member having a first articulating surface and a second member having a second articulating surface. The method includes forming the first component from a stock material including a selected hardness of at least about 45 Rockwell hardness substantially throughout. Also, forming the second component from a stock material having at least about a 45 Rockwell hardness substantially throughout. The first component is finished to define the first articulating surface and the second component is finished to define the second articulating surface. The method also includes implanting the first component and the second component to replace the natural joint such that the first articulating surface and the second articulating surface interact to substantially mimic the natural joint.

A fifth of the various embodiments includes a method of implanting a prosthetic including a metal-on-metal surface to substantially eliminate wear imperfections on the metal surfaces and substantially eliminate wear debris after implantation. The method includes selecting a hardness of a metal alloy. A first prosthetic member, including a first exterior surface, and a second prosthetic component, including a second exterior surface, are formed from the selected alloy. The method also includes implanting the first prosthetic component and the second prosthetic component in the body such that a portion of the first exterior surface of the first prosthetic component interacts with the second exterior surface of the second prosthetic component after implantation.

A sixth of the various embodiments includes a prosthetic, to replace a portion of an anatomy, formed of a metal. The prosthetic includes a first member defining a first articulation surface. An implantable member extends from the first member. The first member includes at least about a 45 Rockwell hardness.

Further areas of applicability will become apparent from the detailed description provided hereinafter. It should be understood that the detailed description and various examples, while indicating the various embodiments, are intended for purposes of illustration only and are not intended to limit the scope of the following claims.

### BRIEF DESCRIPTION OF THE DRAWINGS

The present description will become more fully understood from the detailed description and the accompanying drawings, wherein:

Figure 1 is a partially exploded view of an implanted hip prosthetic including a femoral component and an acetabular component;

Figure 2 is a perspective view of an non-work hardened stock member;

Figure 3 is a diagrammatic view of roller hardening the stock member of Figure 2;

Figure 4 is a perspective view of the work hardened stock member of Figure 3;

Figure 5 is a detailed view of a rough formed head component;

Figure 6 is a detailed view of a portion of a work hardened yet unfinished head portion;

Figure 7 is a detailed partial view of a finished work hardened head component; and

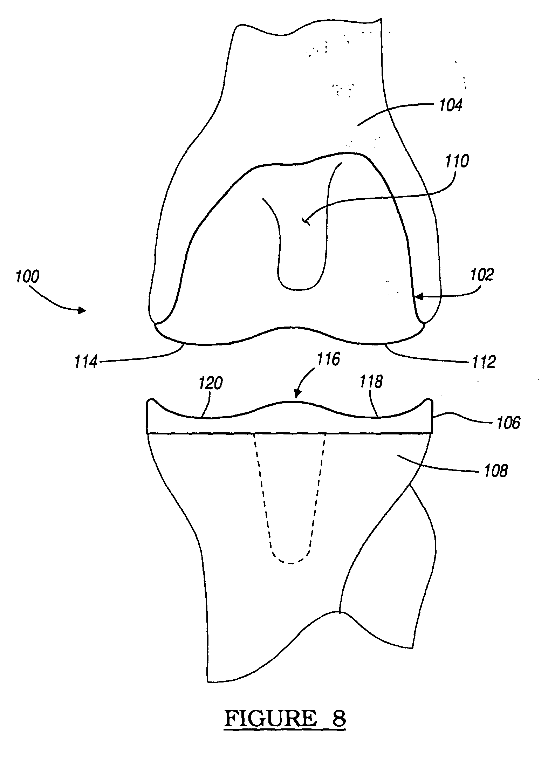

Figure 8 is a simplified view of an implanted prosthetic knee.

### DETAILED DESCRIPTION OF VARIOUS EMBODIMENTS

The following description of various embodiments is merely exemplary in nature and is in no way intended to limit the invention, its application, or uses. The hardened bearing metal-on-metal prosthetic may be used for any prosthetic. Although the following discussion relates specifically to a femoral implant, it will be understood that the same processes, methods and/or materials may be applied to other prosthetics such as a humeral implant or a knee implant. Moreover, although the following discussion relates generally to articulating and bearing prosthetics, it will be understood that the hardened materials may be used in any prosthetics.

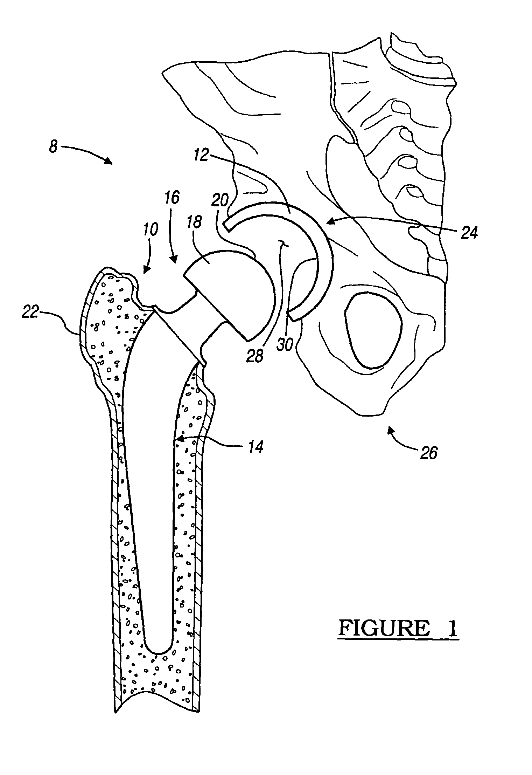

With reference to Figure 1, a bearing metal-on-metal hip joint prosthetic 8 is used to replace a resected hip joint. The hip joint prosthetic 8 includes a femoral component 10 and an acetabular component 12. The femoral component 10 includes a stem portion 14 and a head portion 16. Although illustrated as integral, it will be understood that the femoral component 10 may be modular such that the stem portion 14 can be separate from the head portion 16. The two portions may then be assembled during the implant procedure. The head portion 16 generally defines a ball segment 18 of the hip prosthetic 8 that has a head articulating surface 20. The head articulating surface 20 may be sized depending upon the anatomy of the patient and the desired range of motion after implantation. The femoral components 10 is implanted into a femur 22 by inserting and fixing the stem portion 14 in the femur 22 and orienting the head portion 16 for desired placement after final implantation of the hip prosthetic 8.

The femoral prosthetic 10 generally mates with the acetabular prosthetic 12 which is implanted into a reamed acetabulum 24 of a pelvis 26. The acetabular prosthesis 12 defines a socket or cup including a concave portion 28. The exterior of the concave portion 28 defines an acetabular articulating surface 30. Once implanted, the head articulation surface 20 articulates on the acetabular articulating surface 30. Both the femur 22 and the acetabulum 24 may be prepared using generally known methods.

In the hip prosthetic 8, no separate bearing component is included between the acetabular component 12 and the head articulating surface 20 of the femoral component 10. Therefore, the head articulating surface 20 and the acetabular articulating surface 30 must be prepared so that substantial wear debris or wear does not occur. Producing the articulating surfaces 20, 30 which are particularly hard so that they are not easily scratched or worn when abrading on another surface is one method to produce a prosthetic with good longevity. The articulating surfaces 20, 30 may be hardened at two different occasions. A first occasion includes hardening the stock metal from which the head portion 18 and the acetabular implant 12, which define the articulating surfaces 20, 30, are formed. The stock material can be hardened through any generally known technique as long as a selected hardness is reached. A second occasion is when a rough formed head portion 18 and acetabular components 12 may be hardened after they are rough formed. In this case, the specific articulating surfaces 20, 30 are hardened to the selected hardness after being rough formed into the respective components. Increasing the hardness of the articulating surfaces 20, 30 decreases wear and wear debris material after implantation. A hardness of the articulating surfaces 20, 30 above about forty-five (45) Rockwell hardness shows substantial decrease of wear and wear debris after implantation.

### Example 1: Hardening the Stock Material:

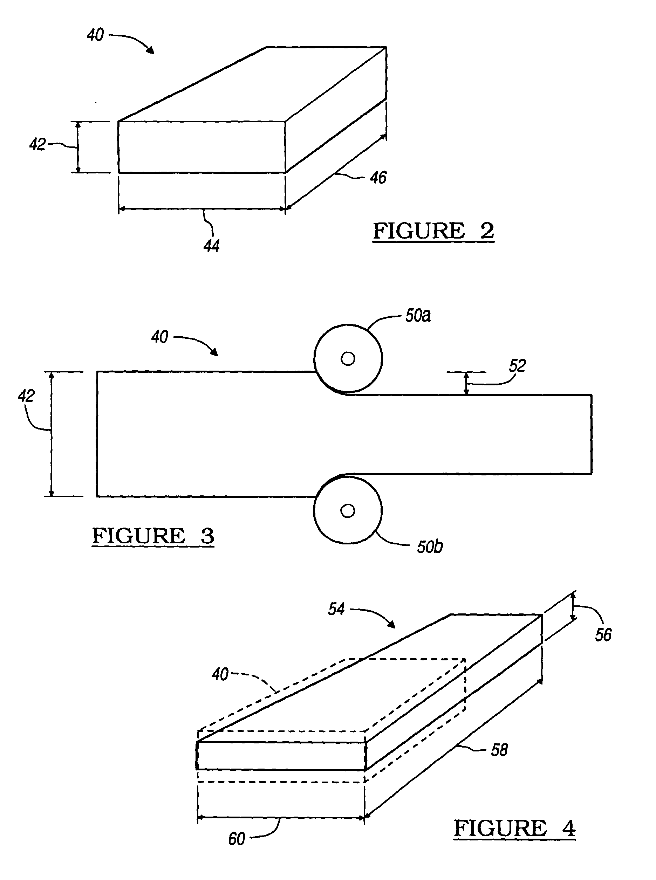

With reference to Figure 2, prosthetics, such as the hip prosthetic 8, are generally formed from a stock bar member 40. The stock member 40 may be any appropriate bio-compatible material but generally includes an alloy of cobalt, chromium, and molybdenum (CoCrMo). The unhardened stock member 40 generally includes a first set of unhardened dimensions. Generally, the dimensions include an unhardened height 42, width 44, and depth 46. Work hardening can then be performed on the non-work hardened stock member 40 through any generally known hardening methods. For example, the material may be shot peened, roller hardened, or laser shocked to induce the desired hardness. Generally, however, to induce enough hardness throughout the entire stock member 40, an intense physical working such as roller working is used.

When performing roller hardening, any one of the three unwork hardened dimensions 42, 44, 46 may be reduced. With reference to Figure 3, the stock member 40 is fed through rollers 50a and 50b to reduce the non-work hardened height dimension 42 by at least the work hardening dimension 52. Therefore, with reference to Figure 4, a work hardened stock member 54 is produced. The work hardened stock member 54 has new dimensions including a work hardened height 56, a work hardened length 58 and a work hardened width 60. Although the stock member 40 was specifically work hardened in the height dimension, each dimension of the stock member may be increased. The non-work hardened stock member 40 is shown in phantom lines for comparison to the work hardened stock member 54. It will be understood that the exact dimensions of either the non-work hardened stock member 40 or the work hardened stock member 54 depend upon the material and the amount of work hardening required to produce the selected hardness in the work hardened stock member 54.

Once the work hardened stock member 54 is formed, it includes a inherent hardness which any of the later produced components from the work hardened stock member 54 will also include. Therefore, if the work hardened stock member 54 includes a Rockwell hardness of at least 45, prosthetic portions formed from the work hardened stock member 54 will also include that same Rockwell hardness.

Non-work hardened stock member 40 may include any generally appropriate stock material form. For example, the non-work hardened stock member 40 may be cast or wrought before being work hardened. Nevertheless, it is the additional work hardening, such as that illustrated in Figure 3, that induces the desired hardnesses in the work hardened stock member 54. This allows for the prosthetic components to be formed from the work hardened stock member 54 to reduce wear after implantation.

Moreover, producing the work hardened stock member 54 reduces later manufacturing steps required to produce the hip prosthetic 8. Specifically, because the work hardened stock member 54 includes the required hardness property, the later formed hip prosthetic 8 can be formed and machined to its finished shape without any additional coatings or work hardening required to produce the selected hardness. Therefore, later manufacturing steps and time are reduced by initially forming the work hardened stock member 54. In addition, because the entire work hardened stock member 54 includes the selected hardness, it is ensured to be present at the articulating surfaces after the hip prosthetic 8 is formed. Simply, the prosthetic is formed from the hardened stock member 54 that includes the selected hardness. No further coatings, hardenings, or surface treatments are needed to ensure the selected hardness is at the articulating surface.

### Example 2: Hardening a Rough Formed Prosthetic:

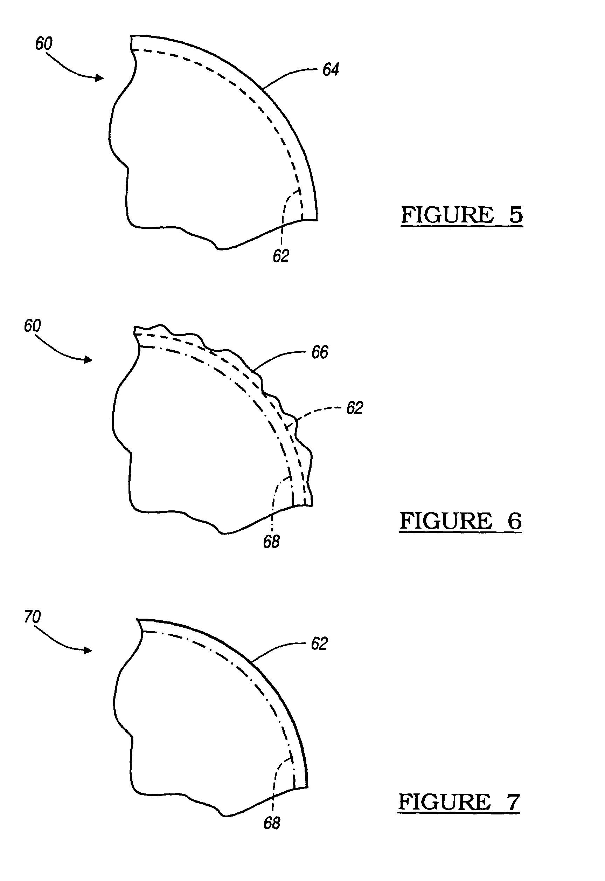

With reference to Figure 5, a detailed portion of an unfinished or rough worked head member 60 is illustrated. The unfinished head member 60 includes a final or finished selected surface dimension 62 and an unfinished surface dimension 64. Generally, the unfinished surface dimension 64 is greater than the finished dimension 62. Therefore, the unfinished head member 60 must be finished in some method to reduce the unfinished dimension 64 to the finished selected dimension 62.

When the unfinished head member 60 is formed from a non-work hardened stock member, such as the non-work hardened stock member 40, the unfinished head member 60 can be work hardened by generally known methods. For example, the unfinished head member 60 can be hardened with shot peening or laser shot peening to increase the hardness of the surface.

With reference to Figure 6, once the rough formed head 60 has been hardened, such as through shot peening, the shot peened surface 66 can be finished. The hardened unfinished head portion 60 includes a hardening interface dimension or transition zone 68 after work hardening. The hardening interface dimension 68 is generally below the finished dimension 62 which defines the finished head portion after the finished dimension 62 has been achieved. It will also be understood, that the hardening interface dimension 68 is not necessarily a distinctly defined line or area. That is, the interface dimension 68 may include a short distance over which the hardness of the material transitions from the work hardened hardness, that is the hardness of the surface 62 to the hardness of the substrate material.

A finished head 70 is finished so that the finished dimension 62 is substantially defined by the exterior of the finished head member 70. As illustrated, the hardened interface 68 is below the finished surface 62. The area between the hardened interface 68 and the finished surface 62 is generally termed a hardened region. The hardened region is at least 0.25 mm thick. The work hardening hardens the hardened region and also forms the transition zone 68. Specifically, the hardness of the material gradually changes from a substantially hardest area at the finished surface 62 to a least hardest area, the hardness of the substrate. The transition zone 68 is at least about 0.25 mm below the finished surface 62. Between the finished surface 62 and the transition zone 68, the hardness of the material gradually decreases to the hardness of the substrate material.

Therefore, the finished surface 62 includes the hardness that was produced by the work hardening that was performed on the unfinished head member 60. The hardening generally produces a phase transformed region between the interface 68 and the finished surface 62. This phase transformed region increases the hardness of the material above the hardness of the non-work hardened material. Generally, the work hardened finished surface 62 has a hardness of at least about 45 Rockwell hardness. Again, any appropriate method may be used to work the rough formed head portion 60.

Although the above examples relate specifically to the formation of the head portion of a hip prosthetic 8, it will be understood that the acetabular articulating surface 30 can be formed in a similar manner. For example, the acetabular component 12 can be formed from the work hardened stock member 54. Therefore, the acetabular articulating surface 30 would include the hardness formed into the work hardened stock member 54. Regardless, the hip prosthetic 8 includes a head articulating surface 20 and an acetabular articulating surface 30 which are both hardened to a selected hardness. Specifically, the head articulating surface 20 and the acetabular articulating surface 30 are hardened to a Rockwell hardness of at least 48. Therefore, as the head articulating surface 20 articulates against the acetabular articulating surface 30, a generally insignificant amount of wear occurs on either articulating surface 20, 30. Because wear is reduced, the longevity of the hip prosthetic 8 is increased. Reduced wear reduces the necessity of revision procedures because of excessive wear on an implanted prosthetic.

In addition, the increased hardness of the articulating surfaces 20, 30 reduces the wear debris which may form in other hip prosthetics. Because the wear debris which may form it reduces the abrasive materials which might otherwise be present in the hip prosthetic 8 and would otherwise increase wear. Again, this increases the longevity of the hip prosthetic 8 and reduces the possibility of requiring a revision procedure.

In addition, the metal-on-metal bearing prosthetic eliminates the need for an additional bearing component. Specifically, because of the hardened articulating surfaces 20, 30 an additional component that is adapted to absorb the wear and friction in the hip prosthetic 8 is not necessary. Therefore, the hardened articulating surfaces 20, 30 of the hip prosthetic 8 are able to articulate with substantially no deleterious effects due to the metal-on-metal articulation.

It will be understood that any prosthetic can be formed according to the present disclosure or with variations thereon. Generally, hardening the articulating surfaces of a prosthetic can increase the longevity and reduce the wear and production of wear debris after implantation. Therefore, prosthetics can be formed that have substantially increased life spans. Moreover non-bearing prosthetic may also be formed with hardened surfaces. These may be used to decrease biological wear as well.

Another exemplary prosthetic which may be formed to include hardened articulating or bearing surfaces includes a knee prosthetic 100. The knee prosthetic 100 generally includes a femoral component 102, that is implanted onto a femur 104, and a tibial component 106, that is implanted onto a tibia 108. The femoral component 102 generally includes a patella groove 110, a first condyle 112, and a second condyle 114. The tibial component 106 generally includes a bearing surface 116 that may include a first bearing portion 118 to articulate with the first condyle 112 and a second bearing portion 120 to articulate with the second condyle 114. Both the femoral component 102 and the tibial component 106 may be implanted with generally known procedures. Nevertheless, the respective articulating surfaces 112, 114, 118, 120 may be hardened to include a selected hardness to increase the longevity of the knee prosthetic 100.

As described above, each of the components, the femoral component 102 and the tibial component 106, may be formed entirely of a stock work hardened material, such as the work hardened stock member 54. Therefore, the entire knee prosthetic 100 includes the selected hardness at its surfaces. In this way, the articulating surfaces 112, 114, 118, and 120 all include the selected hardness.

Alternatively, the femoral component 102 and the tibial component 106, may be rough formed with the articulating surfaces 114, 112, 118 and 120 then hardened through generally known work hardening methods. Specifically, the femoral component 102 may be rough formed so that each of the condyles 112 and 114 may be work hardened using generally known methods such as shot peening or laser shot peening. Then the surfaces could be finished to their final specifications before implantation. Therefore, the surfaces that articulate would be sufficiently hardened to achieve reduced wear when articulating against a similarly hardened tibial component 106. Therefore, in a similar manner, the tibial component 106 may be rough formed and the articulating surface 116 can then be work hardened through generally known methods. After work hardening the articulating surface 116, the tibial component 106 can then be finished to final specifications such that the condyles 112 and 114 of the femoral component 102 properly articulate with the first and second articulating surfaces 118 and 120 of the tibial component to substantially imitate the natural joint. Again, it will be understood that the knee prosthetic 100 is merely exemplary of the prosthetics that may be formed using the hardened surfaces.

In addition to hardening articulating surfaces to reduce wear and harmful wear debris, the material from which the various prosthetics are formed may also have inherent properties which reduce the formation of debris that can increase wear in the prosthetics. Specifically, a reduced or low carbon metal alloy can be used to form the joint prosthetics to reduce the possibility of releasing carbides after implantation. One exemplary low carbon material is described in U.S. Patent No. 6,187,045 entitled, "Enhanced Biocompatible Implants and Alloys", incorporated herein by reference. When an alloy including a high amount of carbon is used, carbides can form which are released from the prosthetic and exist as wear debris on the articulating surfaces after implantation. The carbides can wear on the articulating surfaces thereby scratching and increasing the wear of the articulating surfaces and releasing more wear debris. Forming the prosthetics from the material including low carbide content decreases the amount of carbides that can be produced and virtually eliminates wear debris from carbide release.

Combining a low carbide material with the hardened articulating surface 20, 30, 112, 114, 116 can substantially reduce or virtually eliminate wear debris which may form after implantation of the prosthetics 8, 100. Hardening a low carbon material increases the resistance of the articulating surface 20, 30, 112, 114, 116 to attack from friction and other wear forces. Therefore, this reduces the probability that scratches and imperfections may form on the surface. This reduces the overall amount of debris which may release from the prosthetic 8, 100. In addition, the low carbon material decreases the probability of carbides being released from the prosthetics 8, 100. Therefore, the two in combination substantially reduces the possibility of forming wear debris in the prosthetics 8, 100.

The description is merely exemplary in nature and, thus, variations that do not depart from the gist of the disclosure are intended to be within its scope. Such variations are not to be regarded as a departure from the spirit and scope.

## Claims

1. A prosthetic device for implantation to replace a natural joint, having an articulating region, comprising:
a first metal prosthetic portion having a first articulating surface;
a second metal prosthetic portion having a second articulating surface; and
said first articulating surface and said second articulating surface each include at least about a 45 Rockwell hardness;
wherein said first prosthetic portion and said second prosthetic portion are implanted in the natural joint so that said first articulating surface articulates with said second articulating surface;
wherein articulation of said first prosthetic portion and said second prosthetic portion does not produce significant wear within the prosthetic.

2. The prosthetic device of claim 1, wherein,
said first articulating surface and said second articulating surface each include a Rockwell hardness of at least about 50;
said first articulating surface and said second articulating surface articulate while producing substantially little wear interaction.

3. The prosthetic device of claim 1, wherein,
said first prosthetic and said second prosthetic portion are both formed from a metal alloy stock including cobalt, chromium, and molybdenum;
said metal alloy stock having a work hardened hardness to include the selected hardness throughout such that said first articulating surface and said second articulating surface include said selected hardness.

4. The prosthetic device of claim 1, wherein said first articulating surface and said second articulating surface are hardened to said selected hardness by shot peening.

5. The prosthetic device of claim 1, wherein:
said first prosthetic device includes a head portion of a femoral component;
said second prosthetic portion includes an acetabular implant to operably interact with said head portion;
said head portion defining said first articulating surface on an exterior of said head portion; and
said acetabular implant defining a concave interior surface defining said second articulating surface.

6. The prosthetic device of claim 1, further comprising:
a first support member extending from said first articulating surface to support said articulation surface in said natural joint; and
a second support member extending from said second articulating surface to support said second articulating surface in the natural joint;
wherein said first support member and said second support member align said first articulating surface and said second articulating surface to substantially mimic the natural joint.

7. A prosthetic device for implantation into a body, comprising:
a first member defining a first articulating surface;
said first member also defining a first hardness region having a first transition zone at least 0.25 mm below said first articulating surface;
a second member defining a second articulating surface adapted to form an articulating region with said first articulating surface; and
said second member also defining a second hardness region having a second transition zone at least 0.25 mm below said second articulating surface;
wherein said first member and said second member are implanted in the body so that said first articulating surface articulates with said second articulating surface during functioning of the prosthetic device.

8. The prosthetic device of claim 7, wherein,
said first articulating surface and said second articulating surface each include a Rockwell hardness of at least about 50;
said first articulating surface and said second articulating surface articulate while producing substantially little wear interaction.

9. The prosthetic device of claim 7, wherein said first hardness region and said second hardness region each have a hardness of at least about 45 Rockwell hardness;
wherein said transition zone includes a hardness gradient from each of said articulating surfaces to a substrate of said respective members.

10. The prosthetic device of claim 7, wherein,
said first member includes a femoral portion for a knee implant, said femoral portion including a first condyle and a second condyle, wherein said first articulating surface is defined by an exterior of each of said condyles; and
said second member includes a tibial component for the knee implant including a tibial bearing surface, wherein said tibial bearing surface defines said second articulating surface.

11. The prosthetic device of claim 7, wherein,
said first member and said second member are both formed to include a metal alloy stock including cobalt, chromium, and molybdenum;
wherein said first articulating surface and said second articulating surface are hardened to said selected hardness by shot peening sufficient to produce said first hardened region and said second hardened region.

12. A prosthetic to replace a natural joint, the natural joint including a first articulating portion and a second articulating portion, comprising:
a first component adapted to replace the first articulating portion;
a second component adapted to replace the second articulating portion;
said first component and said second component each including an articulating surface to substantially replace the natural joint after implantation; and
said first component and said second component being formed from a material having a hardness greater than about 45 Rockwell hardness;
wherein said articulating surface of each of said first component and said second component include the hardness of said material.

13. The prosthetic of claim 12, wherein:
said first component includes a head portion adapted to replace the head portion of a femur and a stem portion extending from said head portion;
said head portion including an exterior surface defining a first articulating surface;
said second component includes a cup portion to be implanted into a prepared acetabulum; and
said cup defines a concave interior portion, wherein said concave interior portion defines said second articulating surface.

14. The prosthetic of claim 13, wherein said first articulating surface and said second articulating surface include substantially the hardness as the remainder of said respective components.

15. A method of forming a prosthetic to replace a natural joint, the prosthetic including a first member having a first articulating surface and a second member having a second articulating surface, the method comprising:
forming the first component from a stock material including a selected hardness of at least about 45 Rockwell hardness substantially throughout;
forming the second component from a stock material having at least about a 45 Rockwell hardness substantially throughout;
finishing the first component to define the first articulating surface;
finishing the second component to define the second articulating surface; and
implanting the first component and the second component to replace the natural joint such that the first articulating surface and the second articulating surface interact to substantially mimic the natural joint.

16. The method of claim 15, further comprising:
resecting the natural joint to prepare the natural joint for implantation of the prosthetic;
implanting the first component into a first boney potion of the joint; and
implanting the second component into a second boney portion, such that the first articulating surface and the second articulating surface interact to mimic the natural joint.

17. The method of claim 15, wherein finishing the first component to define the first articulating surface, and finishing the second component to define the second articulating surface does not eliminate the selected hardness from the first articulating surface nor the second articulating surface.

18. A method of implanting a prosthetic including a metal-on-metal surface to substantially eliminate wear imperfections on the metal surfaces and substantially eliminate wear debris after implantation, comprising;
selecting a hardness of a metal alloy;
forming a first prosthetic member including a first exterior surface from the selected alloy;
forming a second prosthetic component including a second exterior surface from the selected alloy; and
implanting the first prosthetic component and the second prosthetic component in the body such that a portion of the first exterior surface of the first prosthetic component interacts with the second exterior surface of the second prosthetic component after implantation.

19. The method of claim 18, wherein selecting the metal alloy includes selecting a metal alloy which includes or can be work hardened to include at least about a 45 Rockwell hardness.

20. The method of claim 18, wherein forming the first prosthetic component and the second prosthetic component includes:
rough forming the first prosthetic component or the second prosthetic component;
work hardening the first exterior surface of the first prosthetic component or the second exterior surface of the second prosthetic component to form a work hardened region; and
finishing the first prosthetic component or the second prosthetic component to include selected tolerances for implantation into the body.

21. A prosthetic, to replace a portion of an anatomy, formed of a metal, comprising:
a first member defining a first articulation surface; and
an implantable member extending from said surface member;
wherein said first member includes at least about a 45 Rockwell hardness.

22. The prosthetic of claim 21, wherein said first member includes said at least about 45 Rockwell hardness after being work hardened.

23. The prosthetic of claim 21, wherein said first member includes said at least about 45 Rockwell hardness throughout.

24. the prosthetic of claim 22, wherein said first member and said implantable member are integral.
